# EUROPÄISCHE PATENTANMELDUNG

(11) **EP 1 449 837 A1**
(43) Veröffentlichungstag der Anmeldung: **25.08.2004**
(21) Anmeldenummer: 04002756.7
(22) Anmeldetag: 07.02.2004
(51) Int. Cl.: C07D 277/80

(54) **Verfahren zur Herstellung von Benzthiazolylsulfenamiden durch Umsetzung von primären Aminen mit Alkalimetallsalzen von Mercaptobenzthiazol in Gegenwart von Wasserstoffperoxid und Alkalimetallhypochlorit**

(30) Priorität: 20.02.2003 DE 10307138
(71) Anmelder: Bayer Aktiengesellschaft, 51368 Leverkusen (DE)
(72) Erfinder: Grabowski, Stefan, Dr., 41539 Dormagen (DE); Felix, Leon, 9140 Temse (BE); Sluyts, Domien, 2940 Hoevenen (BE); Missiaen, Rateik, Dr., 2650 Edegem (BE); Preuss, Reinhard, Dr., 2930 Brasschaat (BE)

(57) **Zusammenfassung**

Die Erfindung betrifft ein Verfahren zur Herstellung von Benzthiazolylsulfenamiden durch Umsetzung von primären Aminen mit Alkalimetallsalzen von Mercaptobenzthiazol in Gegenwart von Wasserstoffperoxid und Alkalimetallhypochlorit. Das erfindungsgemäße Verfahren zeichnet sich insbesondere durch sehr hohe Umsatzraten aus, wobei die erhaltenen Benzthiazolylsulfenamide in hoher Reinheit, hohen Ausbeuten und einer hohen Lagerstabilität - auch bei höheren Temperaturen und hoher Luftfeuchtigkeit - erhalten werden.

## Beschreibung

Die Erfindung betrifft ein Verfahren zur Herstellung von Benzthiazolylsulfenamiden durch Umsetzung von primären Aminen mit Alkalimetallsalzen von Mercaptobenzthiazol in Gegenwart von Wasserstoffperoxid und Alkalimetallhypochlorit. Das erfindungsgemäße Verfahren zeichnet sich insbesondere durch sehr hohe Umsatzraten aus, wobei die erhaltenen Benzthiazolylsulfenamide in hoher Reinheit, hohen Ausbeuten und einer hohen Lagerstabilität - auch bei höheren Temperaturen und hoher Luftfeuchtigkeit - erhalten werden.

Es ist bekannt, Benzthiazolylsulfenamide, die vor allem als Vulkanisationsbeschleuniger in der Kautschuk-verarbeitenden Industrie eingesetzt werden, durch gemeinsame Oxidation von Mercaptobenzthiazolen oder ihren Alkalimetallsalzen und Aminen herzustellen. Als Oxidationsmittel können beispielsweise Chlor, Hypochlörite oder Wasserstoffperoxid eingesetzt werden.

So wird beispielsweise in der europäischen Patentanmeldung 0 180 869-A2, ein Verfahren zur Herstellung von Benzthiazolylsulfenamiden beschrieben, wobei diese durch Oxidation im wässrigen Medium aus Mercaptobenzthiazol oder seinen Salzen und primären Aminen erhalten werden.

Nachteilig bei dem in der genannten europäischen Patentanmeldung beschriebenen Verfahren ist jedoch, dass bei der Oxidation mit Wasserstoffperoxid das eingesetzte Mercaptobenzthiazol nur bis zu 75 % umgesetzt werden kann, um die in den Beispielen der Patentanmeldung beschriebene Reinheit und Ausbeute zu erhalten. Da das Mercaptobenzthiazol nur mit einer relativ niedrigen Umsatzrate umgesetzt werden kann, geht dies jedoch zu Lasten der Wirtschaftlichkeit des in der Europäischen Patentanmeldung beschriebenen Verfahrens.

Nachteilig bei der beschriebenen Oxidation mit Hypochlorit ist die große Menge an Chlorid-Ionen, die bei dem Prozess gebildet wird und entsprechend entsorgt werden muss. Darüber hinaus ist dieses Verfahren mit beträchtlichen Korrosionsproblemen behaftet.

In der US-Patentschrift 5,436,346 wird ebenfalls ein Verfahren zur Herstellung von Benzthiazolysulfenamiden durch Umsetzung von 2-Mercaptobenzthiazol mit primären oder sekundären oder cycloaliphatischen Aminen in Gegenwart von Wasserstoffperoxid als Oxidationsmittel im wässrigen Medium beschrieben.

Nachteilig bei diesem Verfahren ist, dass als Ausgangsprodukte 2-Mercaptobenzthiazole eingesetzt werden, die erst aus den entsprechenden Alkalimetallsalzen hergestellt werden müssen, was natürlich mit entsprechenden Ausbeuteverlusten - bedingt durch Reinigungsverfahren - einhergeht. Darüber hinaus ist die technische Durchführung des in der US-Patentschrift beschriebenen Verfahrens umständlich und daher weniger wirtschaftlich.

Aufgabe der vorliegenden Erfindung ist es daher, ein ökologisch und wirtschaftlich verbessertes Verfahren zur Verfügung zu stellen, das ausgehend von den Alkalimetallsalzen von Mercaptobenzthiazolen zu lagerstabilen (insbesondere bei höheren Temperaturen und hoher Luftfeuchtigkeit) Produkten führt und das es ermöglicht, auch Ausgangsprodukte einzusetzen, die in technischer Qualität zur Verfügung stehen (ca. 93 bis 97 %ig). Darüber hinaus soll das vorliegende Verfahren hohe Umsatzraten bezüglich der eingesetzten Alkalimetallsalze von Mercaptobenzthiazol gewährleisten, verbunden mit einer hohen Reinheit und einer hohen Ausbeute bezüglich der Benzthiazolylsulfenamide.

Es wurde nun überraschend gefunden, dass unter den erfindungsgemäßen Bedingungen Benzthiazolylsulfenamide erhalten werden, die - wie erwähnt - mit hoher Reinheit und großer Lagerstabilität in hohen Ausbeuten verbunden mit hohen Umsatzraten bezüglich des eingesetzten Alkali-Mercaptobenzthiazols anfallen.

Gegenstand der Erfindung ist daher ein Verfahren zur Herstellung von Benzthiazolylsulfenamiden durch Umsetzung von primären Aminen mit Alkalimetallsalzen von Mercaptobenzthiazol in Gegenwart von Wasserstoffperoxid und Alkalimetallhypochloriten, dadurch gekennzeichnet, dass man eine wässrige Lösung eines Metallsalzes von Mercaptobenzthiazol mit primären Aminen in Gegenwart von maximal 90 mol-% Wasserstoffperoxid, bezogen auf das Alkalimetallsalz des eingesetzten Mercaptobenzthiazols, bei einem pH-Wert im Bereich von 10,0 bis 11,0 umsetzt, wobei die Menge an Wasserstoffperoxid innerhalb eines Zeitraumes von ≥2 Stunden zudosiert wird, anschließend zu dem Reaktionsgemisch innerhalb eines Zeitraumes von ≥10 Minuten Alkalimetallhypochlorit zur weiteren Umsetzung der eingesetzten Alkalimetallsalze des Mercaptobenzthiazols zugibt und nach Ende der Umsetzung den pH-Wert des Reaktionsgemisches auf 11,5 bis 12 erhöht, wobei die eingesetzte Menge an primären Aminen 100 bis 500 mol-% und die Summe an eingesetztem Wasserstoffperoxid und eingesetztem Alkalimetallhypochlorit maximal 130 mol-% betragen, bezogen auf die Menge an Alkalimetallsalz des eingesetzten Mercaptobenzthiazols.

Als primäre Amine kommen insbesondere Cyclohexylamin, tert.-Butylamin, tert.-Amylamin und Isopropylamin in Betracht, besonders bevorzugt wird Cyclohexylamin und tert.-Butylamin eingesetzt. Als Alkalimetallsalze von Mercaptobenzthiazol kommen bevorzugt das Natrium- und das Kaliumsalz in Betracht, besonders bevorzugt das Natriumsalz.

Bei dem erfindungsgemäßen Verfahren werden die Alkalimetallsalze des Mercaptobenzthiazols in wässriger Form eingesetzt. Die Konzentration der wässrigen Lösung der Alkalimetallsalze des Mercaptobenzthiazols beträgt üblicherweise 10 bis 60 %, bevorzugt 20 bis 50 %.

Bei dem erfindungsgemäßen Verfahren wird bevorzugt in Gegenwart von 50 bis 90 mol-% Wasserstoffperoxid, ganz besonders bevorzugt 70 bis 90 mol-% Wasserstoffperoxid, bezogen auf die Menge an Alkalimetallsalz des eingesetzten Mercaptobenzthiazols, gearbeitet.

Welche Menge an Wasserstoffperoxid in bevorzugter Weise eingesetzt wird, hängt u.a. von den Reaktionsbedingungen ab und von der Art der eingesetzten Metallsalze des Mercaptobenzthiazols sowie den eingesetzten primären Aminen. Die günstigste Menge kann jeweils durch entsprechende Vorversuche bestimmt werden.

Für das erfindungsgemäße Verfahren ist es von Bedeutung, dass die Menge an Wasserstoffperoxid innerhalb eines bestimmten Zeitraums zudosiert wird: In bevorzugter Weise wird die Menge an Wasserstoffperoxid innerhalb eines Zeitraums von 2 bis 5 Stunden, besonders bevorzugt innerhalb eines Zeitraums von 3 bis 4 Stunden dem Reaktionsgemisch zudosiert. Der Zeitraum der Zudosierung von Wasserstoffperoxid hängt wiederum von den Reaktionsbedingungen sowie von den gewählten Einsatzprodukten ab und kann leicht durch entsprechende Vorversuche bestimmt werden.

Das erfindungsgemäße Verfahren wird in bevorzugter Weise bei einem pH-Wert im Bereich von 10,2 bis 10,8 durchgeführt.

Nachdem die vorgesehene Menge an Wasserstoffperoxid dem Reaktionsgemisch zugegeben wurde, wird anschließend zu dem Reaktionsgemisch innerhalb eines Zeitraums von bevorzugt 15 bis 90 Minuten, ganz besonders bevorzugt 20 bis 60 Minuten Alkalimetallhypochlorit zur weiteren Umsetzung der eingesetzten Alkalimetallsalze des Mercaptobenzthiazols zudosiert.

Als Alkalimetallhypochlorite kommen beispielsweise in Betracht Na-Hypochlorit und K-Hypochlorit, bevorzugt das Natriumhypochlorit.

Nach dem Ende der Umsetzung der eingesetzten Alkalimetallsalze des Mercaptobenzthiazols wird der pH-Wert des Reaktionsgemisches bevorzugt auf 11,6 bis 11,8 erhöht. Die Erhöhung des pH-Werts nach Ende der Umsetzung ist von Bedeutung, um insbesondere lagerstabile Endprodukte zu erhalten.

Die eingesetzte Menge an primären Aminen beträgt bevorzugt 133 bis 400 mol-%, bezogen auf die Menge an Alkalimetallsalz des eingesetzten Mercaptobenzthiazols. Auch hierbei kann die günstigste Menge an einzusetzenden primären Aminen jeweils durch entsprechende Vorversuche bestimmt werden.

Bei dem erfindungsgemäßen Verfahren wird die Menge an eingesetztem Alkalimetallhypochlorit so gewählt, das sich die Summe an eingesetztem Wasserstoffperoxid und eingesetztem Alkalimetallhypochlorit bevorzugt zu 115 mol-% ergänzen. Wird beispielsweise eine Menge an Wasserstoffperoxid von 80 mol-% gewählt, so beträgt die Menge an eingesetztem Alkalimetallhypochlorit maximal 35 mol-%.

Die Konzentration der eingesetzten wässrigen Wasserstoffperoxidlösung beträgt ca. 5 bis 50 %, bevorzugt 5 bis 35 %, insbesondere 5 bis 15 %, die Konzentration der eingesetzten wässrigen Lösung an Alkalimetallhypochlorit beträgt ca. 12 bis 15, bevorzugt 13 bis 14 %.

Das erfindungsgemäße Verfahren kann sowohl kontinuierlich als auch diskontinuierlich dürchgeführt werden, wobei die diskontinuierliche Fahrweise bevorzugt ist.

Das erfindungsgemäße Verfahren wird bei Temperaturen von ca. 35 bis 60°C, bevorzugt 40 bis 55°C durchgeführt.

Nach Ende der Reaktion werden die erhaltenen Benzthiazolylsulfenamide beispielsweise durch Filtration isoliert und das erhaltene Produkt gewaschen und getrocknet.

Wie erwähnt, können bei dem erfindungsgemäßen Verfahren die Salze des Mercaptobenzthiazols in technischer Qualität eingesetzt werden, d.h. in einer Reinheit von größer als 93 %, bevorzugt in einer Reinheit von 93 bis 95 %. Selbstverständlich ist es auch möglich, die Mercaptobenzthiazole in höherer Reinheit einzusetzen, dies geht jedoch dann zu Lasten der Wirtschaftlichkeit des erfindungsgemäßen Verfahrens.

Nach dem erfindungsgemäßen Verfahren werden die Benzthiazolylsulfenamide in Reinheiten von >99 % und in Ausbeuten von >92 % der Theorie erhalten. Dabei betragen die Umsatzraten bezüglich der eingesetzten Alkalimetallsalze des Mercaptobenzthiazols >99 %.

Weiterhin vorteilhaft ist es, dass bei dem erfindungsgemäßen Verfahren nur sehr geringe Mengen an Chlorid-Ionen gebildet werden; wodurch sich das Verfahren besonders umweltfreundlich gestaltet im Vergleich zu Verfahren die als Oxidationsmittel nur Chlor oder nur Natriumhypochlorit einsetzen. Obwohl bei dem erfindungsgemäßen Verfahren überwiegend mit Wasserstoffperoxid oxidiert wird und nur geringere Mengen an Natriumhypochlorit eingesetzt werden, ist es als überraschend zu werten, dass ausgehend von den Alkalimetallsalzen von Mercaptobenzthiazol die Benzthiazolylsulfenamide in solch guten Qualitäten wie beschrieben erhalten werden mit ausgesprochen hohen Ausbeute und hohen Umsatzraten.

### Beispiele

### Beispiel 1

In einen Reaktor wurden 995,94 g einer wässrigen, 20 %igen Lösung von Natrium-2-Mercaptobenzthiazol gegeben und zu dieser wässrigen Lösung 139,35 g Cyclohexylamin (100 %ig) unter intensivem Mischen (Mischleistung 0,4 Watt/l) hinzugefügt. Die Reinheit des eingesetzten Natrium-Mercaptobenzthiazols betrug 95 %. Das Molverhältnis von reinem Nätrium-Mercaptobenzthiazol zu Cyclohexylamin betrug 1 : 1,4 mol; d.h. auf 100 mol-% Natrium-Mercaptobenzthiazol mit einer Reinheit von 95 % wurden 133 mol-% an Cyclohexylamin eingesetzt. Der Reaktor war mit einem Rückflusskühler, einem Thermometer und einer pH-Messelektrode ausgestattet. Mit Hilfe einer 20 %igen Schwefelsäure wurde der pH-Wert des Reaktionsgemisches bei 40°C auf 10,6 eingestellt und die Temperatur während der gesamten Reaktion auf 40°C belassen.

Innerhalb eines Zeitraums von 5 Stunden wurde gleichmäßig 0,821 mol Wasserstoffperoxid (10 %ig) dem Reaktionsgemisch hinzugefügt. Dies bedeutet, dass 78 mol-% an Wasserstoffperoxid auf 100 mol-% an 95 %igem Natrium-Mercaptobenzthiazol eingesetzt wurden.

Nach der Zugabe des Wasserstoffperoxids wurde innerhalb von 38 Minuten 13 %iges Natriumhypochlorit in einer Menge von 202 g dem Reaktionsgemisch hinzugegeben und nach dem Ende der Reaktion durch Zugabe von 50 %igem wässrigen Natriumhydroxid der pH-Wert auf 11,8 erhöht. Die Reaktionsmischung wurde anschließend auf 30°C abgekühlt, filtriert, mit 10 %igem Cyclohexylamin gewaschen, danach nochmals mit Wasser nachgewaschen und das ausgefallene Produkt getrocknet. Erhalten wurden 245,56 g an Benzthiazolylsulfenamid, was eine Ausbeute von 97,76 %, bezogen auf reines Natrium-Mercaptobenzthiazol, bedeutet. Die Umsatzrate betrug 99,4 %, bezogen auf reines NaMBT. Das erhaltene Produkt besaß eine Reinheit von 99,2 %, bestimmt durch Titration nach ASTM D 4936 (MBT).

Bei der Umsetzung betrugt die Summe an eingesetztem Wasserstoffperoxid und eingesetzem Alkalimetallhypochlorit insgesamt 112 mol-%, bezogen auf die eingesetzte Menge an 95 %igem Natrium-Mercaptobenzthiazol.

### Beispiel 2

Es wurde wie unter Beispiel 1 gearbeitet, jedoch mit dem Unterschied, dass 1,06 mol 10 %iges Wasserstoffperoxid (101 mol-%, bezogen auf 100 mol-%iges Natrium-Mercaptobenzthiazol) eingesetzt wurden. Wasserstoffperoxid wurde innerhalb von 4 Stunden bei 40°C Reaktionstemperatur zudosiert. Innerhalb von 10 Minuten wird weiter oxidiert mit 57,8 g einer 13 %igen NaOCl-Lösung.

Die Ausbeute betrug 96,8 %, die Reinheit 99,2 % und die Umsatzrate 99,4 %.

### Beispiel 3

Es wurde wie unter Beispiel 2 gearbeitet, jedoch wurde das Wasserstoffperoxid innerhalb von 3 Stunden bei 40°C Reaktionstemperatur zudosiert. Innerhalb von 12 Minuten wird weiter oxidiert mit 61,6 g einer 13 %igen NaOCl-Lösung.

Die Ausbeute betrug 96,6 %, die Reinheit 98,1.% und die Umsatzrate 99,4 %.

### Beispiel 4

Dieses Beispiel wurde analog Beispiel 1 durchgeführt, jedoch mit 1,10 möl Cyclohexylamin und mit 90 mol-% Wasserstoffperoxid (10 %ig). Das Wasserstoffperoxid wurde innerhalb von 4 Stunden bei 45°C Reaktionstemperatur dem Reaktionsgemisch zugegeben. Innerhalb von 35 Minuten wird weiter oxidiert mit 182 g einer 13 %igen NaOCl-Lösung.

Die Ausbeute betrug 95,2 %, die Reinheit 97,5 % und die Umsatzrate 99,4 %.

### Beispiel 5

Entsprechend dem Beispiel 1 wurde die Reaktion durchgeführt. Eingesetzt wurde Natrium-Mercaptobenzthiazol mit einer Reinheit von 93 %. Die Menge an Cyclohexylamin betrug 1,43 mol, bezogen auf das eingesetzte 100 % reine Natrium-Mercaptobenzthiazol. Es wurden 90 mol-% 10 %iges Wasserstoffperoxid innerhalb von 4 Stunden bei einer Reaktionstemperatur von 40°C dem Reaktionsgemisch zugegeben. Innerhalb von 24 Minuten wird weiter oxidiert mit 12 g einer 13 %igen NaOCl-Lösung.

Die Ausbeute betrug 97,8 %, die Reinheit 98,9 % und die Umsatzrate 99,4 %.

### Vergleichsbeispiel 6

In einen Reaktor wurden 397,89 g einer wässrigen 50 %ige Lösung von Natrium 2-Mercaptobenzothiazol gegeben und zu dieser wässrigen Lösung 187,57 g Cyclohexylamin 100 %ig und 1684 g Wasser unter Mischen hinzugefügt. Die Reinheit des eingesetzte Natrium-Mercaptobenzothiazols betrug 95 %. Anschließend wurden 368,4 g einer 20 %igen Schwefelsäure bei 50°C zum Reaktionsgemisch gegeben.

Innerhalb eines Zeitraums von 2 Stunden wurde gleichmäßig 1 mol Wasserstoffperoxid (30%ig) dem Reaktionsgemisch bei 50°C hinzugefügt. Nach dem Ende der Reaktion wurde 73,68 g einer wässrigen 50 %igen Lösung von Natriumhydoxid zum Reaktionsgemisch gegeben.

Die Reaktionsmischung wurde anschließend 30 Minuten nachgerührt, auf 30°C abgekühlt, filtriert, mit Wasser gewaschen und das ausgefallene Produkt getrocknet.

Erhalten wurde 222,1 g an Benzthiazolsulfenamid was eine Ausbeute von 84 %, bezogen auf reines Natrium-Mercaptobenzothiazol bedeutet.

Das erhaltene Produkt besaß eine Reinheit von 99%, bestimmt durch Titration nach ASTM D 4936.

Die Mutterlauge enthält noch 34,29 g Natrium-mercaptobenzothiazol wodurch die Umsatzrate 81,87 % betrug.

### Vergleichsbeispiel 7

In einen Reaktor wurde 175 g 2-Mercaptobenzothiazol, 500 g Wasser und 118 g Cyclohexylamin 100 % (1,2 mol) gegeben unter intensiven Mischen. Die Reinheit des eingesetzten Mercaptobenzothiazols betrug 95,5 %.

Das Molverhältnis von rein Mercaptobenzothiazol zu Cyclohexylamin betrug 1:1,2 mol. Die feine Suspension von Cyclohexylaminsalz von 2-Mercaptobenzothiazol wurde geheizt bis 50°C.

Innerhalb eines Zeitraums von 5 Stunden wurden unter intensiven Mischen gleichmäßig 1,1 mol Wasserstoffperoxid (12,6 %) den Reaktionsgemisch zugefügt.

Die Reaktionsmischung wurde anschließend 30 min nachgerührt, auf Zimmertemperatur abgekühlt, filtriert, mit 10 %-igen Cyclohexylamin gewaschen, danach nochmals mit Wasser nachgewaschen und das ausgefallene Produkt getrocknet.

Erhalten wurden 250,2 g an, Benzothiazolsulfenamid.

Die Mutterlauge enthält 4,3 g nicht reagiertes 2-Mercaptobenzothiazol.

Die Umsatzrate betrug 97,4 % und die Ausbeute von Cyclohexylsulfenamid 94,8 %.

Das enthaltene Produkt besaß eine Reinheit von 99,1 %, bestimmt durch Titration nach ASTM D 4936.

## Patentansprüche

1. Verfahren zur Herstellung von Benzthiazolylsulfenamiden durch Umsetzung von primären Aminen mit Alkalimetallsalzen von Mercaptobenzthiazol in Gegenwart von Wasserstoffperoxid und Alkalimetallhypochloriten, **dadurch gekennzeichnet, dass** man eine wässrige Lösung eines Metallsalzes von Mercaptobenzthiazol mit primären Aminen in Gegenwart von maximal 90 mol-% Wasserstoffperoxid, bezogen auf das Alkalimetallsalz des eingesetzten Mercaptobenzthiazols, bei einem pH-Wert im Bereich von 10,0 bis 11,0 umsetzt, wobei die Menge an Wasserstoffperoxid innerhalb eines Zeitraumes von≥2 Stunden zudosiert wird, anschließend zu dem Reaktionsgemisch innerhalb eines Zeitraumes, von ≥10 Minuten Alkalimetallhypochlorit zur weiteren Umsetzung der eingesetzten Alkalimetallsalze des Mercaptobenzthiazols zugibt und nach Ende der Umsetzung den pH-Wert des Reaktionsgemisches auf 11,5 bis 12 erhöht, wobei die eingesetzte Menge an primären Aminen 100 bis 500 mol-% und die Summe an eingesetztem Wasserstoffperoxid und eingesetztem Alkalimetallhypochlorit maximal 130 mol-% betragen; bezogen auf die Menge an Alkalimetallsalz des eingesetzten Mercaptobenzthiazols.

2. Verfahren nach Anspruch 1, wobei als primäre Amine Cyclohexylamin, tert.-Butylamin, tert.-Amylamin sowie Isopropylamin oder Mischungen dieser Amine verwendet werden.

3. Verfahren nach einem der Ansprüche 1 bis 2, wobei die Alkalimetallsalze des Mercaptobenzolthiazols in einer 10 bis 60 %ige wässrigen Lösung eingesetzt werden.

4. Verfahren nach einem der Ansprüche 1 bis 3, wobei die Menge an Wasserstoffperoxid innerhalb eines Zeitraumes von 2 bis 5 Stunden zu dem Reaktionsgemisch zudosiert wird.

5. Verfahren nach einem der Ansprüche 1 bis 4, wobei nach Ende der Umsetzung der eingesetzten Alkalimetallsalze des Mercaptobenzthiazols der pH-Wert im Bereich von 11,6 bis 11,8 erhöht wird.

6. Verfahren nach einem der Ansprüche 1 bis 5, wobei das Verfahren diskontinuierlich durchgeführt wird.

7. Verfahren nach einem der Ansprüche 1 bis 6, wobei die Reinheit der Salze des Mercaptobenzthiazols im Bereich von 93 bei 95 % liegen.
